# EUROPEAN PATENT SPECIFICATION

(11) **EP 2 015 800 B1**
(45) Date of publication and mention of the grant of the patent: **16.11.2016**
(21) Application number: 06752449.6
(22) Date of filing: 10.05.2006
(51) Int. Cl.: A61M 5/145

(54) **FRONT-LOADING MEDICAL INJECTOR AND SYRINGES FOR USE THEREWITH**
VON VORNE BELADBARES MEDIZINISCHES INJEKTIONSGERÄT UND SPRITZEN ZUR VERWENDUNG DAMIT
INJECTEUR MÉDICAL À CHARGEMENT FRONTAL ET SERINGUES CORRESPONDANTES

(43) Date of publication of application: 21.01.2009
(73) Proprietor: Bayer Healthcare LLC, Whippany, NJ 07981 (US)
(72) Inventor: REILLY, David, M., Glenshaw, Pennsylvania 15116 (US); TROCKI, Mark, Cheswick, Pennsylvania 15024 (US)
(74) Representative: BIP Patents
(86) International application number: PCT/US2006/017927
(87) International publication number: WO 2007/130061

(56) References cited:
- WO-A-01/37905
- WO-A-97/36635
- US-A- 5 782 815
- US-A1- 2004 116 893
- US-A1- 2004 122 370
- US-B1- 6 582 407

## Description

### BACKGROUND OF THE INVENTION

This invention relates to medical injectors, and syringes, syringe interfaces, syringe adapters and syringe plungers for use therewith. More particularly, the present invention relates to front-loading medical injectors, and syringes, syringe interfaces, syringe plungers and adapters for use with new or existing medical injectors wherein a syringe of special construction is mountable upon and removable from the injectors by a releasable mechanism.

Medical injectors and syringes for injecting contrast media into a patient for imaging biological structures are known in the art. For example, U.S. Patent No. 4,677,980, issued to D. M. Reilly et al. on July 7, 1987, and entitled "Angiographic Injector and Angiographic Syringe for Use Therewith," which is assigned to the same Assignee as the subject application, discloses an angiographic injector apparatus. The apparatus is designed for injecting contrast media into the vascular system of an animal, in which syringes are rear-loaded into a pressure jacket of the injector. More specifically, the apparatus comprises a rotatable turret which carries a pair of the pressure jackets and which is rotatable so that when one of the pressure jackets, into which a syringe has been rear-loaded, is in an injection position, the other pressure jacket is in a position in which an associated syringe can be rear-loaded. Subsequently, when injection of contrast media from the first syringe is completed, the turret is rotated to move the first syringe to an unloading-loading position, with the second pressure jacket and the syringe concurrently being moved into the injection position.

In the apparatus disclosed in the '980 patent, a drive member of the angiographic injector can be drivingly connected to, or disconnected from, a plunger of a syringe at any point along the path of travel of the syringe plunger by a releasable mechanism. However, for the releasable mechanism to correctly operate, the syringe plunger must be properly oriented to mate with the injector piston. Further, during loading of the syringe on the injector, the syringe must be correctly aligned within a respective pressure jacket to allow the syringe plunger and the injector piston to connect to and disconnect from each other.

An improved apparatus over the '980 patent apparatus is disclosed in U.S. Patent No. 5,383,858, issued to D. M. Reilly et al. on January 24, 1995, and entitled "Front-Loading Medical Injector and Syringe for Use Therewith," which is also assigned to the same Assignee as the present application. In the apparatus described in the '858 patent, the syringe is front-loaded onto, in at least one embodiment, a pressure jacket-less injector, overcoming one of the drawbacks of the '980 patent injector apparatus.

The injector described in the '858 patent has a first release mechanism for attaching and releasing the syringe from the injector. In addition, the apparatus includes a second release mechanism that engages and disengages the injector piston from the syringe plunger. Upon rotation of the syringe, the syringe is attached to or released from the injector and, simultaneously, the plunger is attached to or released from the piston. The structure disclosed requires that the syringe be installed on the injector in a specific orientation so that the syringe can releasably engage the injector and, simultaneously, the plunger can releasably engage the piston. In addition, as with the syringe disclosed in the '980 patent, during assembly the syringe plunger must be correctly oriented within the syringe.

Another injector apparatus is disclosed in U.S. Patent No. 5,300,031, issued to C. Neer et al. on April 5, 1994, and entitled "Apparatus for Injecting Fluid into Animals and Disposable Front Loadable Syringe Therefor." The '031 patent discloses various embodiments of a pressure-jacketed injector wherein a syringe is loaded into and removed from an injector pressure jacket through an opening provided in the front end of the pressure jacket. To retain the syringe within the pressure jacket, for example, during an injection operation, the front end of the syringe is locked to the front end of the pressure jacket. To correctly connect the syringe to the pressure jacket, the syringe may only be inserted into the pressure jacket in one orientation.

In each example discussed above, the syringe must be connected to the injector in a specific orientation to assure proper syringe mounting. Proper alignment is required to assure that the syringe may be operated properly during a medical imaging procedure. The required orientation, however, hinders rapid attachment and replacement of the syringe. The required orientation may also increase the manufacturing assembly cost and complexity of the syringe.

WO 97/36635 A and WO 01/37905 A each disclose a syringe for use with an injector comprising the features of the preamble part of claim 1.

Accordingly, while the above injector and syringe apparatuses have proven effective, a need has arisen for a simpler front-loading medical injector. More specifically, to facilitate further the loading operation, a need has arisen for a syringe that can be easily connected to the injector without regard for the specific orientation of the syringe and/or syringe plunger. In addition, to simplify assembly of the syringe components, a need has arisen for a syringe with a plunger that does not need to be oriented in a specific relation to the barrel or base of the syringe. Furthermore, to minimize the time required to prepare an injector for an injection procedure, a need has arisen for injectors providing automated features and/or improved control features.

### SUMMARY OF THE INVENTION

The present invention provides medical injectors and syringes for use therewith which address the needs that have arisen for a simpler injector and syringe system. Specifically, the present invention provides, in one aspect, a syringe interface and a mating syringe that cooperate to allow the syringe to be easily, readily and securely fastened to a medical injector. The syringe need not be oriented in any particular manner before being connected to the injector. In addition, the plunger need not be oriented in any particular manner with respect to the barrel of the syringe. The syringe and plunger both are provided with release mechanisms so that the syringe can be quickly installed on and unloaded from the injector and replaced with a new syringe.

In one aspect, the present invention provides a syringe for use with an injector including a syringe retaining mechanism having a flexible ring. The syringe includes a body having a rearward end and a forward end, a plunger movably disposed within the body and at least one attachment member associated with the body. The attachment member cooperates with the flexible ring of the syringe retaining mechanism to releasably attach the syringe to the injector. The syringe further includes at least one release member associated with the body. The release member is operable to cause deformation of the flexible ring to enable release of the syringe from attachment with the injector upon rotation of the syringe about its axis relative to the injector. The release member is preferably positioned axially forward of the attachment member.

The attachment member can be associated with the rear end of the body. The attachment member can, for example, include a radially outward extending flange encompassing the entire perimeter of the syringe. In another embodiment, the attachment member includes a plurality of radially outward extending flanges positioned around the perimeter of the syringe. The attachment flange (or flanges) can also have a sloped rearward surface to facilitate interaction with the flexible ring of the retaining mechanism.

The release member can, for example, include a plurality of radially outward projecting members that deform the flexible ring upon rotation of the syringe about its axis to a disengagement position. In one embodiment, the attachment member includes a radially outward extending flange encompassing the entire perimeter of the syringe and the projecting members extend radially outward at least the same amount as the attachment member. The projecting members can directly contact the flexible ring to deform the flexible ring.

In another aspect, the present invention provides an injector for injecting fluid from a front-loading syringe mounted thereon. The injector includes a housing, a drive member at least partially disposed within the housing and operable to engage a plunger disposed within the syringe, and a syringe retaining mechanism associated with the housing. The syringe retaining mechanism is operable to seat the syringe upon axially rearward motion of the syringe relative to the retaining mechanism, regardless of the orientation of the syringe about the axis of the syringe. In one embodiment, the retaining mechanism consists essentially of a flexible ring maintained at a fixed axial position within the syringe retaining mechanism.

The present invention, along with the attributes and attendant advantages thereof, will best be appreciated and understood in view of the following detailed description taken in conjunction with the accompanying drawings.

### BRIEF DESCRIPTION OF THE DRAWINGS

The various embodiments of the present invention are described in connection with the figures appended hereto, in which:
Figure 1A illustrates a side view of another embodiment of an injector system of the present invention in a disconnected state;
Figure 1B illustrates a perspective view of the syringe of the injector system of Figure 130A in position to be seated within the syringe interface of the injector system;
Figure 1C illustrates a front, hidden line view of the syringe of Figure 130A rotated about its axis to be in engagement with the flexible ring of the syringe interface after axially rearward seating of the syringe within the syringe interface;
Figure 1D illustrates a front, hidden line view of the syringe of Figure 130A rotated about its axis to be in position for disengagement from the flexible ring of the syringe interface;
Figure 2 illustrates a rear, perspective view of the flexible ring of Figure 103 disconnected from the syringe interface housing;
Figure 3 illustrates a rear, perspective view of the flexible ring of Figure 130 in operative connection with the syringe interface housing;
Figure 4 illustrates an exploded or disconnected perspective view of an embodiment of a piston or drive member of an injector and a cooperating plunger assembly of a syringe;
Figure 5A illustrates a side cross-sectional view and a top, hidden line view of the syringe and the plunger assembly rotated about their common axis to an engagement position;
Figure 5B illustrates a side cross-sectional view and a top, hidden line view of the syringe and the plunger assembly rotated about their common axis to a disengagement position;
Figure 6A illustrates a side cross-sectional view and a top, hidden line view of the plunger assembly rotated about its axis to an engagement position relative to the piston;
Figure 6B illustrates a side cross-sectional view and a top, hidden line view of the plunger assembly rotated about its axis to a disengagement position relative to the piston;
Figure 7A illustrates a side view of a syringe and a connector or syringe interface in which the syringe includes a flexible ring for releasably attaching the syringe to the connector;
Figure 7B illustrates the syringe and connector of Figure 136A in which the connector is shown in a cutaway view and in which the syringe is releasably connected to the connector; and
Figure 7C illustrates the syringe and connector of Figure 136A in which the connector is shown in a cutaway view and in which the syringe has been rotated to place the flexible ring in a stressed state for release of the syringe from the connector.

### DETAILED DESCRIPTION OF THE INVENTION

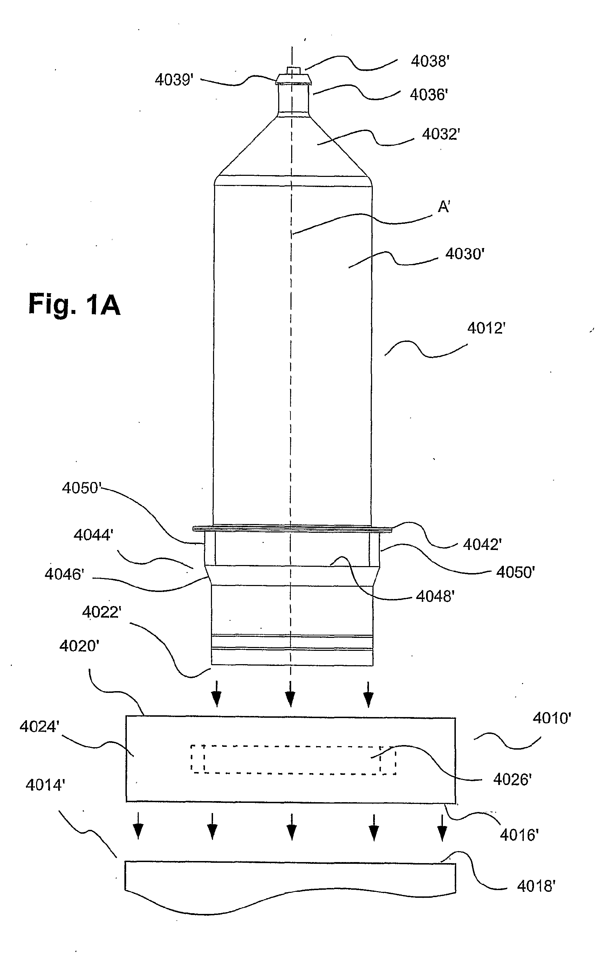

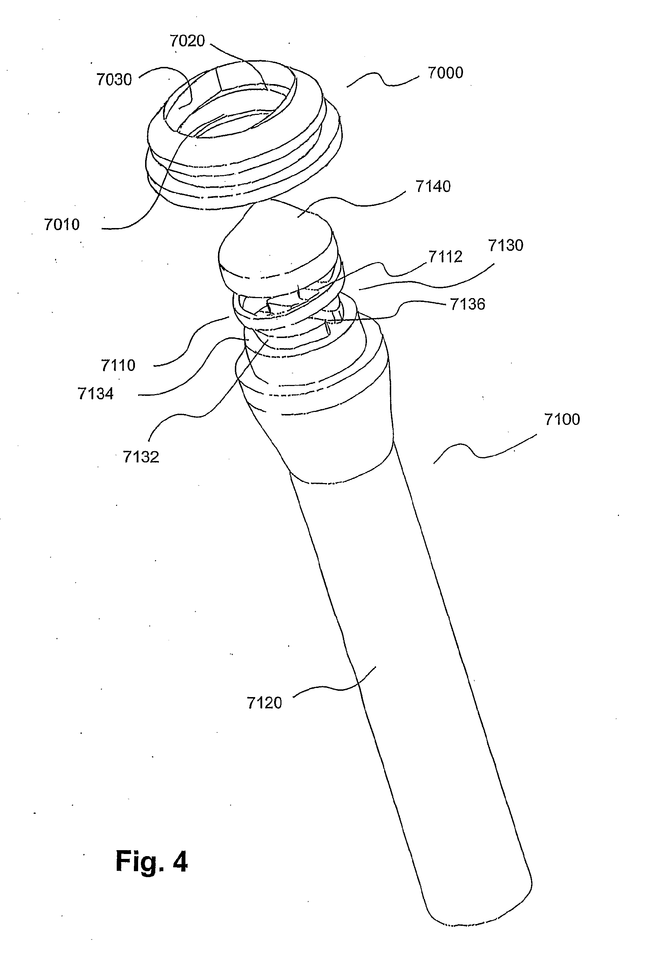

Other embodiments of the present invention are illustrated in Figures 1A through 6B. Of these drawings, Figures 1A through 3 illustrate an embodiment of a syringe interface/connector that releasably secures the syringe to the injector housing. Figures 4 through 6B illustrate an embodiment of an injector piston and syringe plunger interface system/assembly of the present invention that cooperate to axially move the plunger within the syringe.

The injector system includes a syringe interface encompassing a connector 4010' for connecting a syringe 4012' to an injector 4014'. Like syringe interface/connector 4010, syringe interface/connector 4010' provides a mechanism by which a syringe 4012' may be seated quickly relative to an injector 4014' without the requirement for any particular orientation or rotation of the syringe 4012' about its longitudinal axis relative to the injector 4014' during seating. Figure 1A, for example, illustrates syringe interface/connector 4010' (hereinafter, sometimes referred to as connector 4010', for brevity) of the present invention disconnected from injector 4014'. A rear surface 4016' of connector 4010' attaches to a front surface 4018' of injector 4014'. A front surface 4020' of connector 4010' is adapted to receive a rear end 4022' of syringe 4012'. In an alternate embodiment, the connector 4010' maybe integrally formed with the injector 4014'.

Unlike connector housing 4024, the releasable connection mechanism of connector housing 4024', can include only a generally elliptical flexible ring or flex ring 4026', which is disposed at a fixed axial position within connector 4010' near front end 4020'. Although the shape of flex ring 4026' is referred to herein as generally elliptical, it is clear to one skilled in the art that the shape of flex ring 4026' need not precisely follow the path of an ellipsis. Flex ring 4026' operates in generally a similar manner to flex ring 4026 described above to connect syringe 4012' thereto. In the embodiment of Figures 1A though 3, however, other elements or components within connector 4010' designed to cooperate with flex ring 4026' (for example, such as rotating ring 4028 of the embodiment of Figures 55 through 78) to permit release of syringe 4012' from connection with connector 4010' are not required. In that regard, flex ring 4026' and syringe 4012' are adapted to cooperate with one another (preferably without intervening or additional elements or components), as described in greater detail below, to permit connection and release of syringe 4012' to and from connector 4010' (and, accordingly, to and from injector 4014'). In this manner, syringe interface or connector 4010' is substantially simplified in construction and operation as compared to connector 4010. Syringe 4012' is, however, also suitable for releasable connection to interface 4010 of injector 4014.

Syringe 4012' includes a cylindrical body 4030' with a tapering conical portion 4032' at a front end 4034'. Conical portion 4032' is integrally connected to a discharge end 4036'. Discharge end 4036' is provided with a luer lock 4038' that may be connected to a tube (not shown) that is connected ultimately to the patient (also not shown). Syringe 4012' further includes a flange 4039' that can cooperate with a connector as described in U.S. Patent Application Publication No. 2002-0147429, filed on February 20, 2002.

Like syringe 4012, syringe 4012' maybe made from any suitable material, such as a polymeric material. At rear end 4022', syringe 4012'can include a flange 4042', which, when syringe 4012' is connected to connector 4010', helps to prevent contrast medium that may leak from, for example, discharge end 4036' or luer lock 4038', from entering connector 4010'.

A ridge or flange 4044' is integrally formed on syringe 4012' to the rear of flange 4042' toward rear end 4022' of syringe 4012'. In the illustrated embodiment, flange 4044' is formed to be continuous around the perimeter of syringe 4012'. Alternately, as described in connection with syringe 4012, the ridge or flange may be segmented into two or more sections instead of a single continuous member. However, such sections should collectively provide sufficient surface area and strength to retain the syringe 4012' on the injector 4014'.

Similar to ridge 4044, ridge 4044' includes two parts, a sloping section 4046' and a shoulder section 4048' that is essentially perpendicular to the exterior surface of cylindrical body 4030'. Two or more extending tabs or projections 4050' are preferably provided forward of shoulder 4048', between shoulder 4048' and flange 4042'. Tabs 4050' engage flex ring 4026' to release syringe 4012' from connection with connector 4010' as further described below.

As discussed above in connection with connector 4010, to mount syringes of other types on connector or syringe interface 4010' of the present invention, a syringe adapter incorporating the structural components (e.g., ridge 4044', tabs 4050' and/or flange 4042') of the rear end 4022' of syringe 4012' can be fashioned to fit to such a syringe for mounting on the injector of the present invention. To properly engage such a syringe, such an adapter preferably includes structural components complementary to the mounting elements of that syringe.

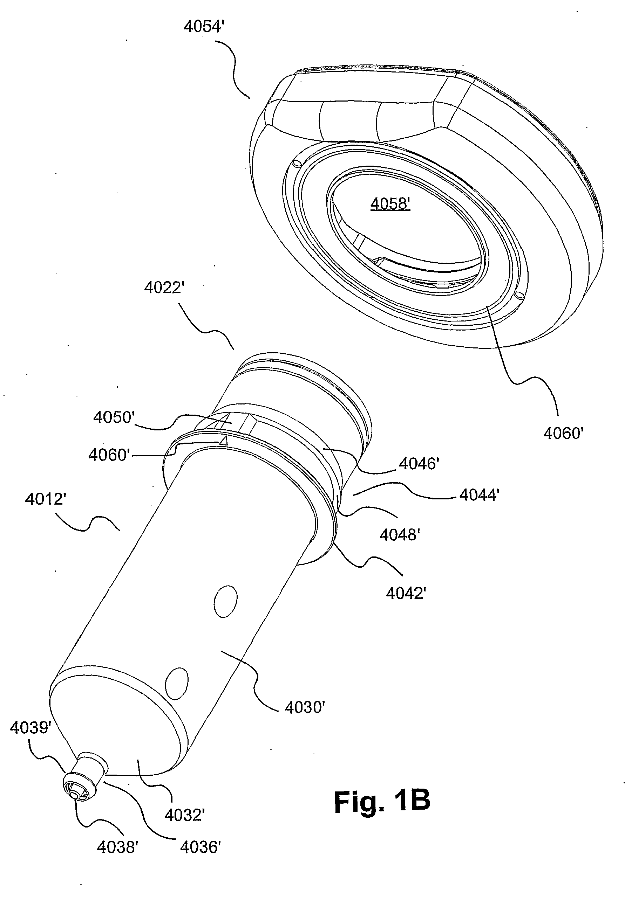

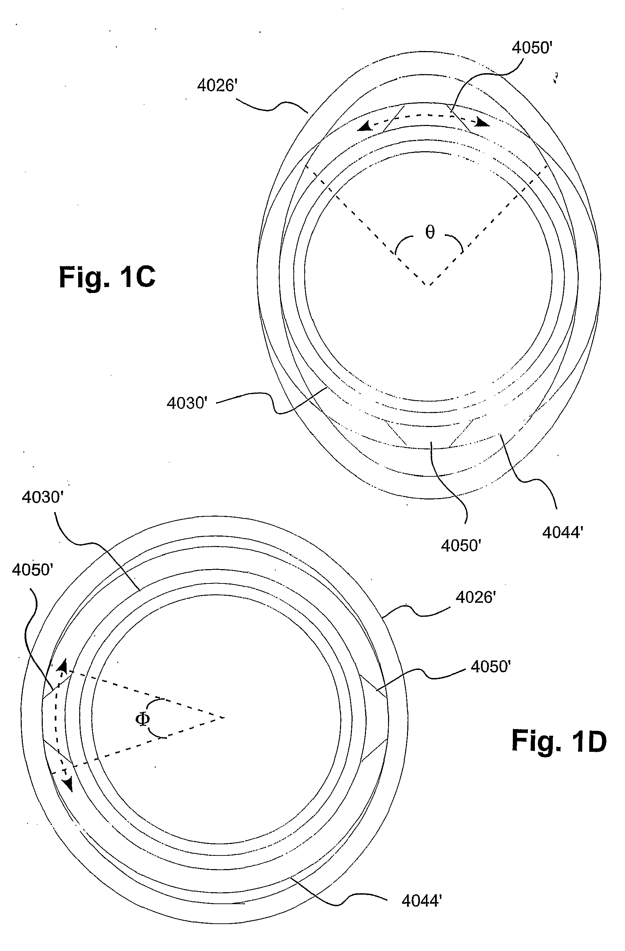

As illustrated in Figures 1, 2 and 3, front plate 4054' includes a hole or opening 4058' therethrough. A lip 4060' extends around the periphery of hole 4058' through front plate 4054'. In one preferred embodiment, when syringe 4012' engages connector 4010', flange 4042' and lip 4060' mate with one another to minimize any leaked contrast medium from entering the interior of connector 4010' through hole 4058'. Alternatively, syringe 4012' may be constructed so that it does not include flange 4042', as would be understood by those skilled in the art. Although, it is advantageous to minimize the ingress of contrast medium into the interior of connector 4010', it is believed that connector 4010' will operate even if fouled with some contrast medium, which is often unavoidable. Flange 4042' can also, however, serve an additional function as a mechanical stop when it engages with front surface 4020' of front plate 4054', ensuring proper axial positioning of syringe 4012' with respect to injector 4014'.

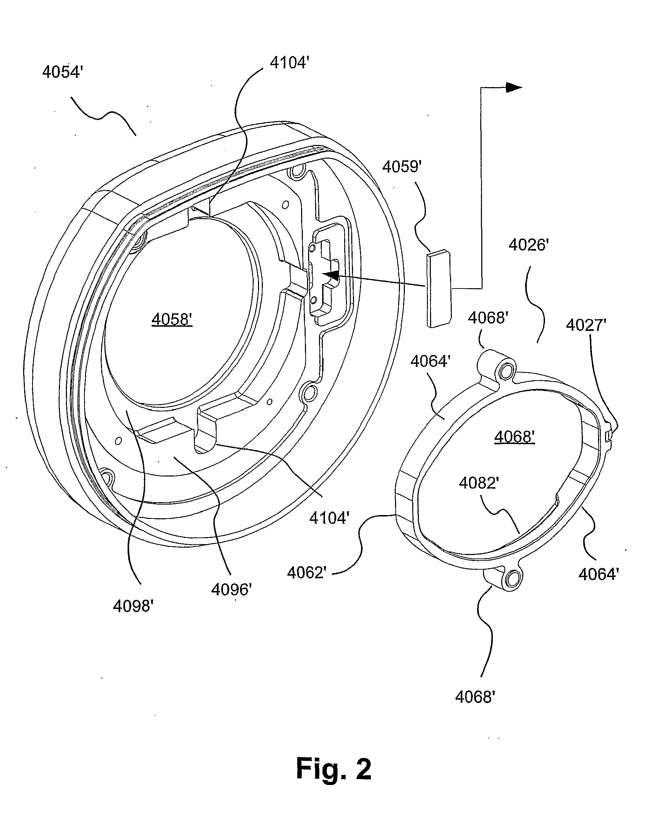

As described above in connection with flex ring 4026, flex ring 4026' may be made from an acetal copolymer or any other suitable material. As illustrated in, for example, in Figure 2, flex ring 4026' can include, on either side, a linear or flattened portion 4062' that is integrally connected to two curved portions 4064'. As shown, flex ring 4026' includes a hole 4068' therethrough. On a front side of flex ring 4026', one or more chamfered surfaces 4082' can be provided to facilitate insertion of rear end 4022' and ridge 4044' of syringe 4012' therethrough.

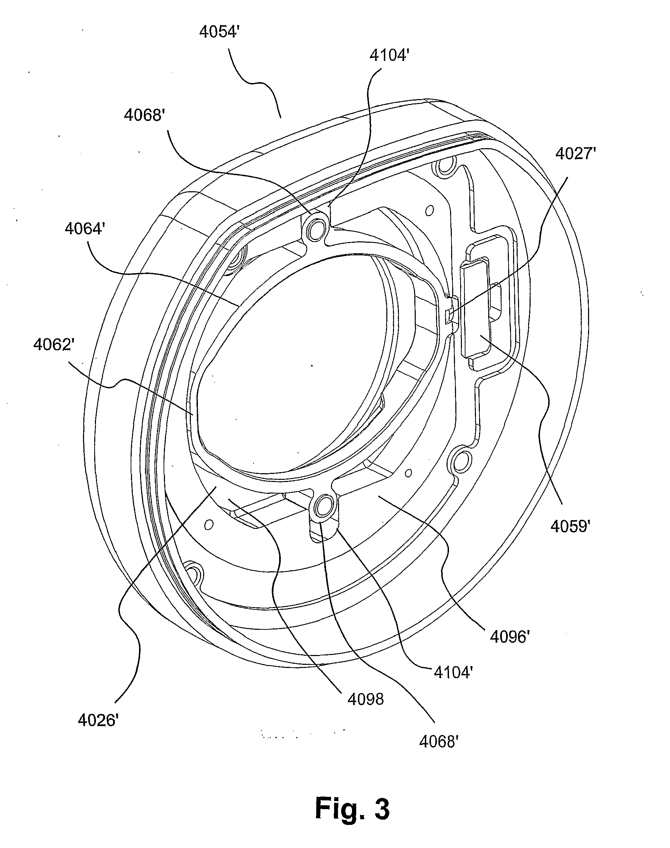

As shown, for example, in Figure 3, a rear surface 4096' of front plate 4054' includes an indentation or recess 4098' that has a shape similar to flex ring 4026'. Two notches 4104' are formed in rear surface 4096' of front plate 4054'. Notches 4104' accommodate extensions 4068'of flex ring 4026'. Indentation 4098' is shaped to be larger than flex ring 4026' and a distance between notches 4104' is greater than a distance between extension 4068 when flex ring 4026' is in a relaxed state. Notches 4104' help to prevent flex ring 4026' from rotating within housing 4024', while permitting flex ring 4026' to expand to its extended state as described below.

To connect syringe 4012' to interface 4010', rearward end 4022' of syringe 4012' is inserted into connector housing 4024' through hole or interface 4058' in front plate 4054'. Flex ring 4026' is maintained or fixed within indentation 4098' formed in rear surface 4096' of front plate 4054' so that extensions 4068 are seated in notches 4104'. When inclined surface 4046' of ridge 4044' of syringe 4012' engages chamfers 4082' on flex ring 4026', ridge 4044' forces flex ring 4026' from its relaxed state (see, for example, Figure 1C) to its extended (or tensioned) state (see, for example, Figure 1D).

After ridges 4044' clear the rear edge of flex ring 4026' and radially outward extending projections or tabs 4050' are rotated out of contact with flex ring 4026' as discussed further below (see, for example, Figure 1C), the elastic nature of flex ring 4026' causes flex ring 4026' to resume its relaxed state. When flex ring 4026' can resume its relaxed state, shoulder 4048' of ridge 4044' engages the rear edge of flex ring 4026'. The syringe 4012' is thereby held in place by flex ring 4026' and cannot be axially removed from connector 4010'. When flex ring 4026' resumes its relaxed state, an audible and/or other signal as described above can be provided to indicate to the operator that the syringe 4012' has been installed on the injector. The audible and/or other signal can, for example, be created mechanically or electronically. In the embodiment of Figures 1A through 3, flex ring 4026' has attached thereto on at least one of generally flat portions 4062' (corresponding generally with the long or major axis of flex member 4026') a magnet 4027' or other element that cooperates with a proximity sensor 4059' (for example, a Hall effect sensor) to determine if flex ring 4026' has returned to its relaxed state, for example, after insertion of syringe 4012'. In that regard, when flex ring 4026' is in its relaxed state, magnet 4027' is in proximity to sensor 4059'. Conversely, when flex ring 4026' is in its extended state, magnet 4027' is moved away from sensor 4059'. Preferably, sensor 4059' is in operative connection with the injector control system to prevent operation of injector 4014' if the flex ring is in its extended state (indicating that syringe 4012' is not properly or fully connected to interface or connector 4010'). Sensor 4059' can also trigger an audible and/or other indication that syringe 4012' is properly connected to connector 4010' or that syringe 4012' is disengaged from connector 4010'.

After proper connection of syringe 4012' to connector 4010', removal of syringe from connector 4010' requires that syringe 4012' be rotated approximately ¼ turn or approximately 90°, as described below. This operation is illustrated, for example, in Figures 1C and 1D. In general, projections 4050' preferably extend radially outward at least to the same extent as ridge or flange 4044'. When syringe 4012' is rotated about its axis to the position illustrated in Figure 1D, projections 4050' abut flex ring 4026' and force flex ring 4026' into its extended position. In this position, syringe 4012' can be moved axially forward relative to connector 4010' so that ridge 4044' passes forward of flex ring 4026' and syringe 4012' is released from connection to connector 4010'.

Similar to the operation of syringe 4012 and connector 4010, syringe 4012' is capable of being fully seated within connector 4010' (that is, moved to its rearwardmost axial position) regardless of the orientation or rotation of syringe 4012' about its axis relative to connector 4010'. As discussed above, removal of the requirement of precise orientation to fully seat a front loading syringe within a syringe interface or connector significantly decreases the difficulty of connecting the syringes of the present invention to the syringe interfaces or connector mechanisms of the present invention as compared to conventional syringes in which, for example, mounting flanges must be accurately aligned with slots formed in the syringe interface to rearwardly seat the syringe.

Unlike syringe 4012, once syringe 4012' is fully rearwardly seated within connector 4010' (or within connector 4010), the operator must ensure that syringe 4012' is rotated about its axis so that projections 4050' move out of contact with flex ring 4026' and flex ring 4026' (or flex ring 4026) is permitted to return to its relaxed state as illustrated, for example, in Figure 1C. Requiring rotation of syringe 4012' to a predetermined position or to within a predetermined range of positions after syringe 4012' is fully rearwardly seated within connector 4010' (or within connector 4010) only minimally increases the operator time required to connect syringe 4012' to connector 4010' as compared to the cooperation of syringe 4012 and connector 4010. However, use of projections 4050' to effect release of syringe 4012' from connection with connector 4010', enables substantial simplification of connector 4010' as compared to connector 4010. As illustrated in Figure 1C, syringe 4012' can be rotated such that projections 4050' are within a range of positions varying over an angle θ of approximately 90° to maintain retaining connection with connector 4010' or with connector 4010. Similarly, as illustrated in Figure 1D, syringe 4012' can be rotated such that projections 4050' are within a range of positions varying over an angle Φ of approximately 10° to effect release of syringe 4012' from connection with connector 4010' or with connector 4010.

Figures 4 through 6B illustrate an embodiment of an injector piston and a cooperating syringe plunger interface assembly 7000 for use in connection with, for example, injector 4014'. In this embodiment, piston 7100 includes a generally elliptical or oval flexible ring 7110 that operates to releasably connect injector piston 7100 to plunger interface assembly 7000. Although the shape of flexible ring 7110 is referred to herein as generally elliptical, it is clear to one skilled in the art that the shape of flexible ring 7110 need not precisely follow the path of an ellipsis. As illustrated in, for example, Figure 133, piston 7100 includes a first or rearward member 7120, which includes a seating 7130 for elliptical flexible ring 7110. Preferably, flexible ring 7110 includes one or more members, such as radially inward extending members 7112, that prevent rotation of flex ring 7110 relative to piston 7100 when flexible ring 7110 is seated in seating 7130. In the embodiment of Figure 133 seating 7130 includes a generally cylindrical, axially forward projecting member 7132 over and around which flexible ring 7110 passes to abut with a generally circular ledge 7134. A slot 7136 is formed in member 7132 into which projections 7112 are seated to prevent rotation of flex ring 7110 relative to piston 7100. Piston 7100 can further include a second or forward member 7140 that attaches to rearward member 7120 as illustrated, for example, in Figures 133 through 135B.

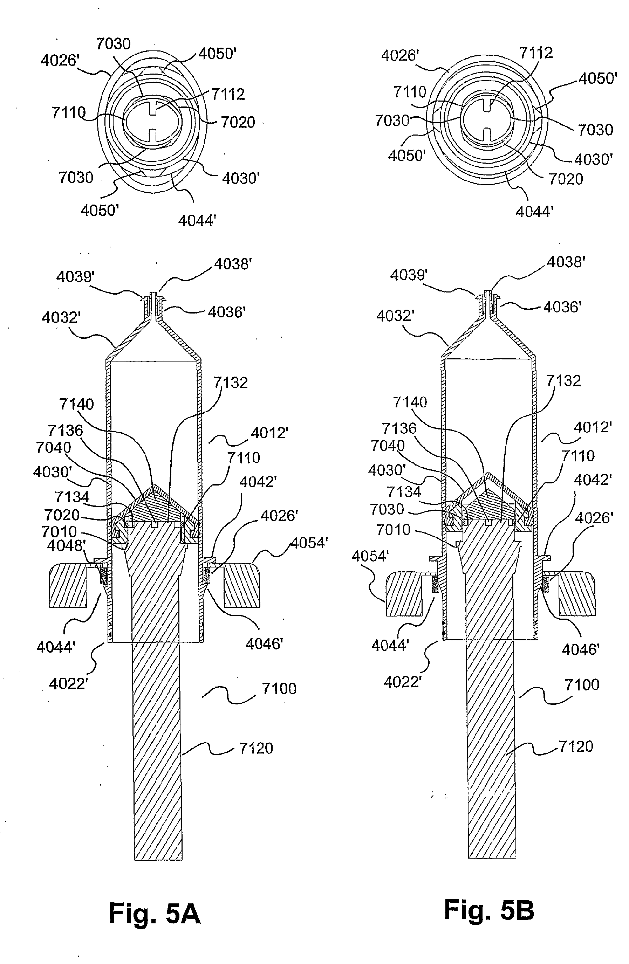

When generally elliptical or oval flexible ring 7110 is seated in seating 7130 as described above and flex ring 7110 is in its relaxed state, a portion of flexible ring 7110 in the general area of the major axis or widest portion of flexible ring 7110 extends beyond the outward edge of ledge 7134 as illustrated, for example, in Figure 5A. As syringe 4012' is moved axially rearward to seat within connector 4010', plunger interface assembly 7000 can be appropriately axially positioned with syringe 4012' and piston 7100 appropriately axially positioned such that the corresponding rearward movement of plunger assembly 7000 with rearward movement of syringe 4012' causes piston 7100 to fully seat within plunger assembly 7000 when syringe 4012' fully seats within connector 4010'. Alternatively, piston 7100 can be seated within plunger assembly 7000 separately from the seating of syringe 4012' within connector 4010'. The relative orientation or rotation of plunger assembly 7000 about its axis is preferably irrelevant to the ability of piston 7100 to seat within plunger assembly 7000.

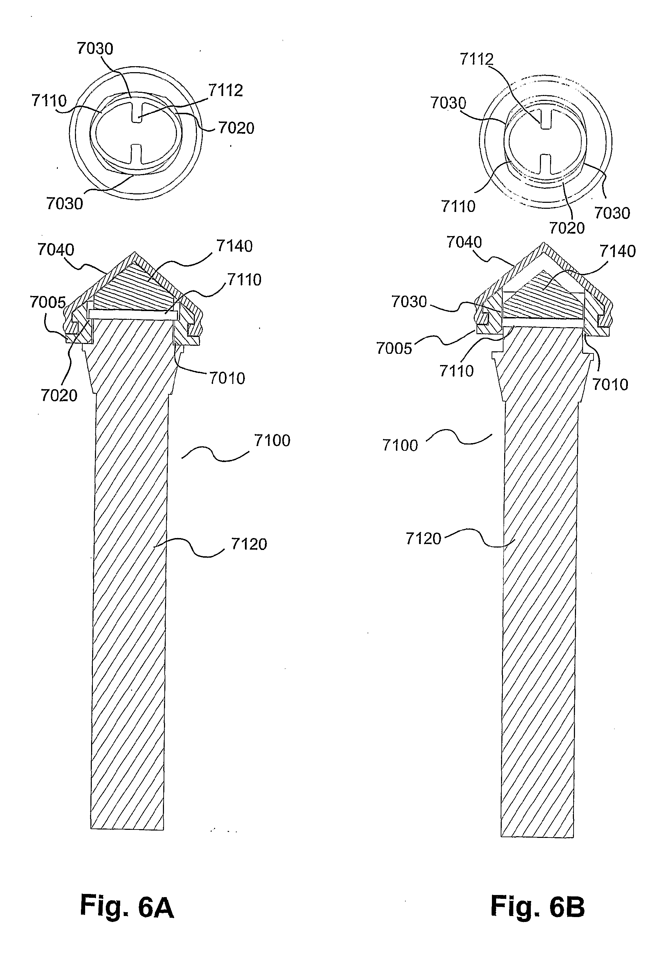

In the embodiment of Figures 4 through 6B, plunger assembly includes a base 7005 having a chamfered surface 7010 on a reward end thereof that causes flexible ring 7110 to deform to a stressed state (see Figures 134B and 135B) as necessary to allow flexible ring 7110 to pass forward of ledge 7020 formed around the inner surface of plunger base 7005. After flexible ring 7110 is forward of ledge 7020, flexible ring 7110 is free to return to its relaxed state if plunger base 7005 is rotated about its axis to the position illustrated in Figures 5A and 6A.

In that regard, forward of ledge 7020 the generally cylindrical interior wall of plunger base 7005 includes radially inward extending or flattened portions 7030. When plunger assembly 7000 is rotated about its axis so that flattened portions 7030 are generally aligned with the long or major axis of flexible ring 7110, abutment of flattened portions 7030 with flexible ring 7110 forces flexible ring 7110 into its stressed state as illustrated in Figures 5B and 6B. In this state, piston 7100 can be moved axially rearward relative to plunger assembly 7000 (or plunger assembly 7000 moved axially forward relative to piston 7100) such that plunger assembly 7000 can be disconnected from piston 7100. Conversely, when flattened portions 7030 are rotated to a position approximately ¼ turn or 90° from alignment with the long or major axis of flexible ring 7110 (to generally align flattened portions 7030 with the short or minor axis of flexible ring 7110), flexible ring 7110 will be in its relaxed state as illustrated in Figures 5A and 6A so that a rearward surface of flex ring 7110 abuts ledge 7020 and rearward motion of piston 7100 will cause plunger assembly 7000 to move rearward within syringe 4012' (to, for example, draw fluid into syringe 4012'.

Preferably, plunger assembly 7000 is oriented or rotated about its axis within syringe 4012' so that the position of flattened or radially inward projecting portions 7030 are in general alignment with radial outward extending projections 4050' of syringe 4012' as illustrated in Figures 5A and 5B. Plunger assembly 7000 is also preferably positioned within syringe 4012' so that plunger 4012' will not rotate about its axis relative to syringe 4012'. Friction between an elastomeric plunger cover 7040 and the inner wall of the syringe barrel can, for example, prevent rotation of plunger assembly 7000 relative to syringe 4012'. In this manner, after syringe 4012' is moved axially rearward to be seated within connector 4010', as syringe 4012' is rotated to the position described in connection with Figures 1C to be fully engaged within connector 4010', plunger assembly 7000 will be rotated to be in full engagement with piston 7100 as illustrated in, for example, Figure 5A. Likewise, when syringe 4012' is rotated ¼ turn for disengagement with connector 4010' as illustrated, for example, in Figure 5B, flattened portions 7030 of base 7005 will be generally aligned with the long or major axis of flexible ring 7110 as described above, and piston 7100 can be disengaged from plunger assembly 7000.

As clear to one skilled in the art from the above discussion, an elliptical or oval flexible ring or flex ring as discussed above can also be positioned within a plunger assembly and the piston can include a cooperating seating in an injector piston and syringe plunger interface system/assembly of the present invention. Such an embodiment can operate in the same manner in which syringe 4012' collects to and disconnects from connector 4010'.

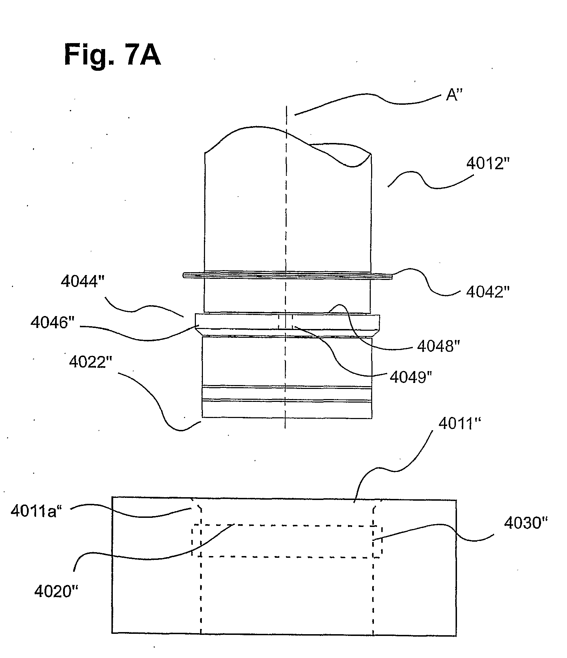

Similarly, Figures 7A through 7C illustrate another embodiment of a syringe 4012" (shown in portion) interface/connector 4010" that releasably secures syringe 4012" to the injector housing (not shown in Figures 7A through 7C) such as injector housing 4014'. Syringe 4012" and interface 4010" cooperate in much the same manner as piston 7100 and plunger assembly 7000 described above. In that regard, syringe 4012" includes flex ring 4044" seated within a recess 4048 formed in a rearward portion of syringe 4012". A rearward portion 4046" of flex ring 4044" can be sloped or beveled to facilitate cooperation with connector 4010" as described below.

In that regard, to connect syringe 4012" to connector 4010", syringe 4012" is inserted into opening or passage 4011". A forward end 4011a" of opening or passage 4011" can be sloped or chamfered to facilitate passing of flex ring 4044" into opening 4011". Syringe 4012" is moved rearward relative to connector 4010" until flex ring 4044" passes beyond ledge 4020" in connector 4010". Syringe 4012" can include flange 4042" to, as discussed above, limit leakage of injection fluids into the injector and/or to assist in proper axial positioning of syringe 4012".

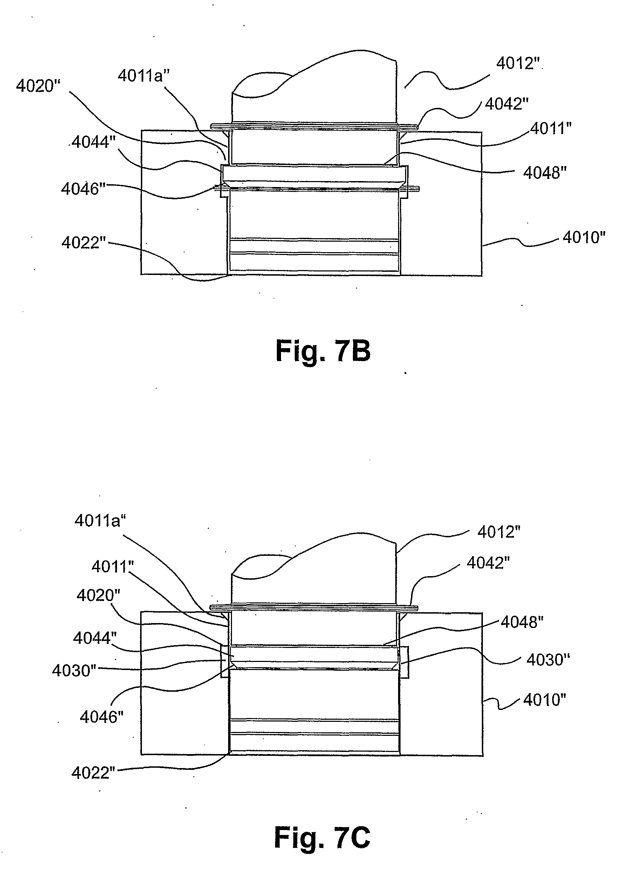

Similar to the design of plunger base 7005, rearward of ledge 4020", the generally cylindrical interior wall of passage 4011" includes radially inward extending or flattened portions 4030". When syringe 4012" is rotated about its axis so that flattened portions 4030" are generally aligned with the long or major axis of flexible ring 4044", abutment of flattened portions 4030" with flexible ring 4004" forces flexible ring 4044" into its stressed state as illustrated in Figure 7C. In this state, syringe 4012" can be moved axially relative to connector 4010" such that syringe 4012" can be disconnected from connector 4010". Conversely, when syringe 4012" and flex ring 4044" are rotated to a position approximately ¼ turn or 90° from the above position such that generally flattened portions 4030" are generally aligned with the short or minor axis of flexible ring 4044", flexible ring 4044" will be in its relaxed state as illustrated in Figure 7B so that a forward surface of flex ring 4044" abuts ledge 4020" and forward axial movement/disconnection of syringe 4012" is prevented. Rotation of flex ring 4044" relative to syringe 4012" is preferably prevented, for example, by including abutting elements 4049" (see Figure 7A) on flex ring 4044" to prevent such relative rotation. Such abutting elements can, for example, operate in the manner similar to extending elements 7112 of flex ring 7110.

The foregoing description and accompanying drawings set forth the preferred embodiments of the invention at the present time. Various modifications, additions and alternative designs will, of course, become apparent to those skilled in the art in light of the foregoing teachings without departing from the scope of the disclosed invention. For example, the respective mating connection and release mechanisms on the injectors and the syringes described above may be interchanged. The scope of the invention is indicated by the following claims rather than by the foregoing description. All changes and variations that come within the meaning and range of equivalency of the claims are to be embraced within their scope.

## Claims

1. A system comprising a syringe (4012', 4012") and an injector (4014') having a syringe retaining mechanism for retaining said syringe (4012', 4012"), the syringe (4012', 4012") comprising:
a body (4030') comprising a rearward end (4022', 4022") and a forward end;
a plunger (7000) movably disposed within the body (4030');
at least one attachment member (4044', 4048") associated with the body (4030'); and
at least one release member (4050', 4030");
**characterized in that**
the syringe retaining mechanism includes a flexible ring (4026', 4044"),
the at least one attachment member (4044', 4048") cooperates with the flexible ring (4026', 4044") of the syringe retaining mechanism to releasably attach the syringe (4012', 4012") to the injector (4014'); and
the at least one release member (4050', 4030") is operable to cause deformation of the flexible ring (4026', 4044") to enable release of the syringe (4012', 4012") from attachment with the injector (4014') upon rotation of the syringe (4012', 4012") about its axis relative to the injector (4014').

2. The system of claim 1 wherein the at least one release member (4050') is associated with the body (4030') of the syringe (4012').

3. The system of claim 1 or 2 wherein the at least one release member (4050') is positioned axially forward of the at least one attachment member (4044').

4. The system of anyone of claims 1 - 3 wherein the at least one attachment member (4044') comprises a radially outward extending flange (4048') encompassing the entire perimeter of the syringe (4012').

5. The system of claim 4 wherein the extending flange (4048') has a sloped rearward surface (4046') to facilitate interaction with the flexible ring (4026') of the retaining mechanism.

6. The system of anyone of claims 1 - 3 wherein the at least one attachment member (4044') comprises a plurality of radially outward extending flanges (4048) positioned around the perimeter of the syringe (4012').

7. The system of anyone of claims 1 - 6, further comprising a flange member (4042', 4042") associated with the body (4030') and adapted to contact a corresponding surface of the injector (4014') when the syringe is releasably engaged therewith, the flange member being positioned axially forward of the at least one release member.

8. The system of claim 7 wherein the flange member (4042', 4042") is adapted to substantially prevent fluid from entering the interior of the injector (4014').

9. The system of claim 7 or 8 wherein contact of the flange member (4042', 4042") with the corresponding surface of the injector (4014') is an indication of proper axial positioning of the syringe with respect to the injector for releasable engagement of the syringe of the injector.

10. The system of anyone of claims 1 - 7 wherein the at least one attachment member (4044', 4048") is associated with the rear end of the body (4030').

11. The system of anyone of claims 1 - 10 wherein the at least one release member includes a plurality of radially outward projecting members (4050') that deform the flexible ring (4026') upon rotation of the syringe (4012') about its axis to a disengagement position.

12. The system of claim 11 wherein the at least one attachment member comprises a radially outward extending flange (4048') encompassing the entire perimeter of the syringe (4012') and the projecting members (4050') extend radially outward at least the same amount as the attachment member (4044').

13. The system of claim 11 or 12 wherein the projecting members (4050') directly contact the flexible ring (4026') to deform the flexible ring.

14. The system of anyone of claims 1 - 13 wherein the plunger (7000) releasably engages a drive member of the injector (4014') via a flexible ring (7110).

15. The system of any of the preceeding claims, the injector (4014') further comprising:
a housing;
a drive member at least partially disposed within the housing and operable to engage the plunger (7000) disposed within the syringe (4012', 4012"); wherein
the syringe retaining mechanism is associated with the housing and operable to seat the syringe (4012', 4012") upon axial rearward motion of the syringe relative to the syringe retaining mechanism regardless of the orientation of syringe about the axis of the syringe, the syringe retaining mechanism consisting essentially of the flexible ring (4026', 4044") maintained at a fixed axial position within the syringe retaining mechanism.

16. The system according to claim 15, **characterized in that** the drive member comprises a flexible ring (7110) disposed thereon operable to engage the plunger (7000) disposed within the syringe (4012', 4012"), the flexible ring being in a first state adapted to engage the plunger and form a connection therewith when the plunger is rotated about its axis to a first position, the flexible ring deforming to a second position adapted to enable release of the plunger when the plunger is rotated about its axis to a second position.

## Patentansprüche

1. System, umfassend eine Spritze (4012', 4012") und einen Injektor (4014') mit einem Spritzenhaltemechanismus zum Halten der Spritze (4012', 4012"), wobei die Spritze (4012', 4012") Folgendes umfasst:
einen Körper (4030'), der ein hinteres Ende (4022', 4022") und ein vorderes Ende umfasst, einen beweglich im Körper (4030') angeordneten Kolben (7000),
mindestens ein Anbringglied (4044', 4048"), das dem Körper (4030') zugeordnet ist, und
mindestens ein Freigabeglied (4050', 4030"),
**dadurch gekennzeichnet, dass**
der Spritzenhaltemechanismus einen flexiblen Ring (4026', 4044") aufweist,
das mindestens eine Anbringglied (4044', 4048") mit dem flexiblen Ring (4026', 4044") des Spritzenhaltemechanismus zusammenwirkt, um die Spritze (4012', 4012") freigebbar am Injektor (4014') anzubringen, und
das mindestens eine Freigabeglied (4050', 4030") dahingehend betätigbar ist, eine Deformation des flexiblen Rings (4026', 4044") zu veranlassen, um bei Drehung der Spritze (4012', 4012") um ihre Achse bezüglich des Injektors (4014') die Freigabe der Spritze (4012', 4012") aus der Anbringung an den Injektor (4014') zu ermöglichen.

2. System nach Anspruch 1, wobei das mindestens eine Freigabeglied (4050') dem Körper (4030') der Spritze (4012') zugeordnet ist.

3. System nach Anspruch 1 oder 2, wobei das mindestens eine Freigabeglied (4050') axial vor dem mindestens einen Anbringglied (4044') positioniert ist.

4. System nach einem der Ansprüche 1 - 3, wobei das mindestens eine Anbringglied (4044') einen sich radial nach außen erstreckenden Flansch (4048`) umfasst, der den gesamten Umfang der Spritze (4012') umgibt.

5. System nach Anspruch 4, wobei der sich erstreckende Flansch (4048') eine geneigte hintere Fläche (4046') hat, um das Zusammenwirken mit dem flexiblen Ring (4026') des Haltemechanismus zu ermöglichen.

6. System nach einem der Ansprüche 1 - 3, wobei das mindestens eine Anbringglied (4044') eine Vielzahl von sich radial nach außen erstreckenden Flanschen (4048) umfasst, die um den Umfang der Spritze (4012') herum positioniert sind.

7. System nach einem der Ansprüche 1 - 6, ferner umfassend ein Flanschglied (4042', 4042"), das dem Körper (4030') zugeordnet und geeignet ist, eine entsprechende Fläche des Injektors (4014') zu kontaktieren, wenn die Spritze freigebbar damit in Eingriff steht, wobei das Flanschglied axial vor dem mindestens einen Freigabeglied positioniert ist.

8. System nach Anspruch 7, wobei das Flanschglied (4042', 4042") geeignet ist, im Wesentlichen zu verhindern, dass Fluid in das Innere des Injektors (4014') eintritt.

9. System nach Anspruch 7 oder 8, wobei Kontakt des Flanschglieds (4042', 4042") mit der entsprechenden Fläche des Injektors (4014') ein Hinweis auf die richtige axiale Positionierung der Spritze bezüglich des Injektors für den freigebbaren Eingriff der Spritze des Injektors ist.

10. System nach einem der Ansprüche 1 - 7, wobei das mindestens eine Anbringglied (4044', 4048") dem hinteren Ende des Körpers (4030') zugeordnet ist.

11. System nach einem der Ansprüche 1 - 10, wobei das mindestens eine Freigabeglied eine Vielzahl von radial nach außen vorragenden Gliedern (4050') aufweist, die bei Drehung der Spritze (4012') um ihre Achse in eine Ausrückposition den flexiblen Ring (4026') deformieren.

12. System nach Anspruch 11, wobei das mindestens eine Anbringglied einen sich radial nach außen erstreckenden Flansch (4048') umfasst, der den gesamten Umfang der Spritze (4012') umgibt, und sich die vorragenden Glieder (4050') mindestens genauso weit wie das Anbringglied (4044') radial nach außen erstrecken.

13. System nach Anspruch 11 oder 12, wobei die vorragenden Glieder (4050') den flexiblen Ring (4026') direkt kontaktieren, um den flexiblen Ring zu deformieren.

14. System nach einem der Ansprüche 1 - 13, wobei der Kolben (7000) ein Antriebsglied des Injektors (4014') über einen flexiblen Ring (7110) freigebbar in Eingriff nimmt.

15. System nach einem der vorhergehenden Ansprüche, wobei der Injektor (4014') ferner Folgendes umfasst:
ein Gehäuse,
ein Antriebsglied, das mindestens teilweise im Gehäuse angeordnet und dahingehend betätigbar ist, den in der Spritze (4012', 4012") angeordneten Kolben (7000) in Eingriff zu nehmen, wobei der Spritzenhaltemechanismus dem Gehäuse zugeordnet und dahingehend betätigbar ist, die Spritze (4012', 4012") bei axialer nach hinten gehender Bewegung der Spritze bezüglich des Spritzenhaltemechanismus aufzulagern, unabhängig von der Ausrichtung der Spritze um die Achse der Spritze, wobei der Spritzenhaltemechanismus im Wesentlichen aus dem flexiblen Ring (4026', 4044") besteht, der in einer festgelegten axialen Position im Spritzenhaltemechanismus gehalten ist.

16. System nach Anspruch 15, **dadurch gekennzeichnet, dass** das Antriebsglied einen darauf angeordneten flexiblen Ring (7110) umfasst, der dahingehend betätigbar ist, den in der Spritze (4012', 4012") angeordneten Kolben (7000) in Eingriff zu nehmen, wobei der flexible Ring in einem ersten Zustand ist, in dem er geeignet ist, den Kolben in Eingriff zu nehmen und eine Verbindung damit zu bilden, wenn der Kolben um seine Achse in eine erste Position gedreht wird, wobei sich der flexible Ring zu einer zweiten Position deformiert, in der er geeignet ist, die Freigabe des Kolbens zu ermöglichen, wenn der Kolben um seine Achse in eine zweite Position gedreht wird.

## Revendications

1. Système comprenant une seringue (4012', 4012") et un injecteur (4014') comportant un mécanisme de rétention de seringue pour retenir ladite seringue (4012', 4012"), la seringue (4012', 4012") comprenant :
un corps (4030') comprenant une extrémité arrière (4022', 4022") et une extrémité avant ;
un piston (7000) disposé mobile à l'intérieur du corps (4030') ;
au moins un élément de fixation (4044', 4048") associé au corps (4030') ; et
au moins un élément de débrayage (4050', 4030"), **caractérisé en ce que** :
le mécanisme de rétention de seringue comprend un anneau flexible (4026', 4044") ;
ledit élément de fixation (4044', 4048") coopère avec l'anneau flexible (4026', 4044") du mécanisme de rétention de seringue pour fixer amovible la seringue (4012', 4012") à l'injecteur (4014') ; et
ledit élément de débrayage (4050', 4030") sert à provoquer une déformation de l'anneau flexible (4026', 4044") pour permettre de libérer la seringue (4012', 4012") de sa fixation à l'injecteur (4014') lors de la rotation de la seringue (4012', 4012") autour de son axe par rapport à l'injecteur (4014').

2. Système selon la revendication 1, dans lequel ledit élément de débrayage (4050') est associé au corps (4030') de la seringue (4012').

3. Système selon la revendication 1 ou 2, dans lequel ledit élément de débrayage (4050') est placé axialement en avant dudit élément de fixation (4044').

4. Système selon l'une quelconque des revendications 1 à 3, dans lequel ledit élément de fixation (4044') comprend un rebord (4048') s'étendant radialement vers l'extérieur et entourant la totalité du périmètre de la seringue (4012').

5. Système selon la revendication 4, dans lequel le rebord d'extension (4048') comporte une surface arrière (4046') inclinée pour faciliter l'interaction avec l'anneau flexible (4026') du mécanisme de rétention.

6. Système selon l'une quelconque des revendications 1 à 3, dans lequel ledit élément de fixation (4044') comprend une pluralité de rebords (4048) s'étendant radialement vers l'extérieur et placés autour du périmètre de la seringue (4012').

7. Système selon l'une quelconque des revendications 1 à 6, comprenant en outre un élément de collerette (4042', 4042") associé au corps (4030') et apte à entrer en contact avec une surface correspondante de l'injecteur (4014') quand la seringue est en prise amovible avec celui-ci, l'élément de collerette étant placé axialement en avant dudit élément de débrayage.

8. Système selon la revendication 7, dans lequel l'élément de collerette (4042', 4042") est apte à pratiquement empêcher du fluide de pénétrer à l'intérieur de l'injecteur (4014').

9. Système selon la revendication 7 ou 8, dans lequel le contact de l'élément de collerette (4042', 4042") avec la surface correspondante de l'injecteur (4014') est une indication de mise en place axiale correcte de la seringue par rapport à l'injecteur pour enclencher amovible la seringue à l'injecteur.

10. Système selon l'une quelconque des revendications 1 à 7, dans lequel ledit élément de fixation (4044', 4048") est associé à l'extrémité arrière du corps (4030').

11. Système selon l'une quelconque des revendications 1 à 10, dans lequel ledit élément de débrayage comprend une pluralité d'éléments (4050') en saillie radiale vers l'extérieur qui déforment l'anneau flexible (4026') lors de la rotation de la seringue (4012') autour de son axe vers une position de désaccouplement.

12. Système selon la revendication 11, dans lequel ledit élément de fixation comprend un rebord (4048') s'étendant radialement vers l'extérieur et entourant la totalité du périmètre de la seringue (4012') et les éléments en saillie (4050') s'étendent radialement vers l'extérieur d'au moins la même quantité que l'élément de fixation (4044').

13. Système selon la revendication 11 ou 12, dans lequel les éléments en saillie (4050') sont en contact direct avec l'anneau flexible (4026') pour le déformer.

14. Système selon l'une quelconque des revendications 1 à 13, dans lequel le piston (7000) entre en prise amovible avec un élément d'entraînement de l'injecteur (4014') par l'intermédiaire d'un anneau flexible (7110).

15. Système selon l'une quelconque des revendications précédentes, l'injecteur (4014') comprenant en outre :
un boîtier ;
un élément d'entraînement disposé au moins partiellement à l'intérieur du boîtier et servant à entrer en prise avec le piston (7000) disposé à l'intérieur de la seringue (4012', 4012"),
dans lequel le mécanisme de rétention de seringue est associé au boîtier et sert à asseoir la seringue (4012', 4012") lors d'un mouvement axial vers l'arrière de la seringue par rapport au mécanisme de rétention de seringue quelle que soit l'orientation de la seringue autour de l'axe de la seringue, le mécanisme de rétention de seringue consistant essentiellement en l'anneau flexible (4026', 4044") maintenu dans une position axiale fixe à l'intérieur du mécanisme de rétention de seringue.

16. Système selon la revendication 15, **caractérisé en ce que** l'élément d'entraînement comprend un anneau flexible (7110) disposé sur lui et servant à entrer en prise avec le piston (7000) disposé à l'intérieur de la seringue (4012', 4012"), l'anneau flexible étant dans un premier état apte à entrer en prise avec le piston et constituer une liaison avec celui-ci quand on fait tourner le piston autour de son axe jusqu'à une première position, l'anneau flexible se déformant jusqu'à une seconde position apte à permettre de libérer le piston quand on fait tourner le piston autour de son axe jusqu'à une seconde position.
